# EUROPEAN PATENT SPECIFICATION

(11) **EP 3 903 659 B1**
(45) Date of publication and mention of the grant of the patent: **06.12.2023**
(21) Application number: 20172198.2
(22) Date of filing: 29.04.2020
(51) Int. Cl.: A61B 1/00, A61B 1/05, A61B 46/13

(54) **STERILE COVER FOR A MEDICAL IMAGING DEVICE AND STERILE MEDICAL IMAGING SYSTEM**
STERILE ABDECKUNG FÜR EINE MEDIZINISCHE BILDGEBUNGSVORRICHTUNG UND STERILES MEDIZINISCHES BILDGEBUNGSSYSTEM
COUVERCLE STÉRILE POUR UN DISPOSITIF D'IMAGERIE MÉDICALE ET SYSTÈME D'IMAGERIE MÉDICALE STÉRILE

(43) Date of publication of application: 03.11.2021
(73) Proprietor: BHS Technologies GmbH, 6020 Innsbruck (AT)
(72) Inventor: CAPELLI, Mark, 6020 Innsbruck (AT); SANTEK, Michael, 6091 Götzens (AT)
(74) Representative: Prüfer & Partner mbB Patentanwälte · Rechtsanwälte

(56) References cited:
- EP-A1- 2 796 082
- JP-A- H04 329 510
- JP-A- H07 275 185
- JP-A- 2010 088 552
- US-A1- 2016 213 250

## Description

The present invention relates to a sterile cover for a medical imaging device and a sterile imaging system with such cover, in particular, a microscope.

The use of medical imaging devices in a sterile environment requires a sterile handling of such medical imaging device. Accordingly, medical imaging devices are usually covered by a foils and a sterile cover, wherein the sterile cover is adapted to at least partially enclose the imaging portion of the medical imaging device. Presently, such sterile cover is pushed over the imaging portion of a medical imaging device and is affixed thereto by frictional locking due to pressing forces. The fixing portion of the medical imaging device is provided by parallel surfaces to allow a secure frictional locking. However, parallel surfaces may not always be desired, e.g. in view of a medical microscope with inclined objectives, wherein the housing should also provide inclined surfaces to reduce the outer potentially interfering contour. In such event, surfaces deviating from a parallel arrangement may not qualify to provide a secure frictional locking.

In the field of endoscopes, JP HO7 275185 A discloses a sheath device for an endoscope capable of maintaining the antifogging effect of an observation window part by easily and inexpensively renewing an antifogging treatment means installed to the cover glass in this observation window part by constitution relatively simple in the structure over the entire part in the case of removing water drops and stains by spraying a washing liquid to the observation window part. This sheath device for the endoscope includes a sheath into and from which the endoscope insertion part is inserted and removed, a cover member which is disposed at the front end of this sheath, covers at least the observation window part disposed at the front end of the endoscope insertion part, has a transparent part facing at least the observation window part and subjected to an antifogging treatment in this transparent part and a nozzle for washing which is disposed at this cover member and sprays fluid to the outside surface of the transparent part facing the observation window part.

Accordingly, it is an object of the present invention to provide a sterile cover that enhances the freedom of design of a medical imaging device while still allowing a secure fastening of a sterile cover.

The object is solved by a sterile cover for a medical imaging device according to claim 1 and a sterile medical imaging system according to claim 9. Further aspects of the present invention are subject of the dependent claims.

According to one aspect, a sterile cover for a medical imaging device, comprises a frame member configured to cover an imaging front side of the medical imaging device, wherein a side intended to face the imaging front side of the medical device is a facing side of the frame member, and at least one transparent cover portion within the frame member arranged to allow at least imaging by the medical imaging device when the frame member is attached to the medical imaging device, wherein the frame member comprises at least two attachment portions configured to provide a positive-fit connection with corresponding attachment portions of the medical imaging device.

The sterile cover may therefore be affixed to the medical imaging device by a positive-locking or form-fit connection instead of only providing a frictional locking. However, such frictional locking may still be provided to support the positive-fit connection. The terms positive-fit, positive-locking or form-fit may be used synonymously. The respective attachment portions of the sterile cover may not only allow the use of inclined fixing portions by the medical imaging device but may also provide an enhanced freedom of design of the outer surfaces as long as the medical imaging device provides at least respective counter connection portions for engagement with the attachment portions in terms of a positive-fit connection. Furthermore, the proposed positive-fit connection may be less sensitive to manufacturing tolerances than a frictional locking.

Due to the at least two attachment portions the frame member may be less likely to be loosely affixed to the medical imaging device or prone to tilt.

As another advantage, the sterile cover may be affixed to and detached from the medical imaging device without the use of any tools due to the positive-fit connection.

Advantageously, the at least two attachment portions are provided on two opposing positions on the facing side of the frame member.

The likelihood of the frame member or the sterile cover, respectively, to tilt is thereby further decreased. Furthermore, by providing the at least two attachment portions on the facing side of the frame member further reduces potential interfering contours during the sterile handling of the medical imaging device with the sterile cover affixed thereto.

Two opposing attachment portions may also support a parallel attachment of the sterile cover on the medical imaging device. A parallel attachment may reduce the likelihood of the sterile cover being bent or otherwise distorted and therefor the risk of the breakage of the transparent cover portion.

Further, the frame member further comprises at least one guiding member protruding from the facing side of the frame member intended to be received by a recess on the imaging front side of the medical imaging device.

The guiding member may support the attachment process by its guiding function in terms of at least indicating a proper orientation. In particular, the guiding member provides at least a length protruding from the facing side of the frame member that allows the guiding member to be received by a corresponding recess of the medical imaging device before or when the attachment portions are in engagement with corresponding attachment portions of the medical imaging device.

In some embodiments, the at least two attachment portions are configured as resilient snap-fit members, preferably protruding from a periphery of the frame member in a direction extending from the facing side of the frame member.

Resilient snap-fit members may be easily implemented and may only require corresponding recesses and/or projections provided by the medical imaging device for a positive-fit connection. In particular, if such snap-fit members protrude from a periphery of the frame member in a direction extending from the facing side of the frame member, the medical device may not have to provide corresponding positive-fit connection members or portions on the imaging front side but on the peripheral surface with respect to the imaging front side. Accordingly, the imaging front side of the medical device may be unaffected by the positive-fit connection and the medical imaging device may also be retrofitted more easily.

Alternatively or in addition, the attachment portions may be configured as bayonet connectors, tongue and groove members or the like.

Advantageously, the snap-fit members are configured to provide a gap between respective snaps of the snap-fit members and the imaging front side of the medical imaging device in parallel to the facing side of the frame member, preferably less than 4 mm, when the medical imaging device extends outwardly with a positive convergence angle from the imaging front side.

In other words, the distance between two opposing snaps is greater than the imaging front side of the medical device when the sterile cover is oriented to be attached to the medical imaging device. Accordingly, the snap-fit members are not elastically deformed at the beginning of the attachment procedure but in the course of moving the sterile cover further in an attachment direction, i.e. moving the respective snaps beyond the imaging front side.

Due to the gap, the required elastic deformation may be reduced and thus, the choice of materials and designs may be enhanced.

However, as the size of the gap and the convergence angle affect the moving distance of the sterile cover to provide sufficient elastic deformation to achieve a snap-fit locking, it may be desirable to limit such moving distance by limiting the gap. Advantageously, the gap is limited to less than 4 mm, in particular, of a convergence angle of about 84° to 87°.

Preferably, a ratio of a protruding length of the at least one guiding member and a protruding length of the snap-fit members protruding from the facing side of the frame member to the respective ends of snaps opposite to the facing side is less than 1, preferably between 0,41 and 0,74.

With a protruding length of the guiding member or guiding members being less than the protruding length of the snap-fit members, the guiding member may be likely to interfere with an imaging component or any other component or contour of the medical imaging. Specifically, a ratio of 0,41 to 0,74 has been identified to reduce the protruding length of the guiding member while still allowing sufficient guidance.

Furthermore, a ratio of a cross section perpendicular to the protruding length of the at least one guiding member and a corresponding cross-section of the recess on the imaging front side of the medical imaging device may be less than 1, preferably between 0,15 and 0,9.

Due to the cross-section of the guiding member being less than the one of the corresponding recess on the imaging front side, the guiding member may be easily introduced into the recess. Additionally, the configuration may be less sensitive to manufacturing tolerances and the like. To enhance such effects, a ratio of 0,9 or less may be advantageous. However, a ration less than 0,15 may result in an insufficient guiding of the guiding member within the recess and the likelihood of tilting may increase.

In some embodiments, at least one of the at least two attachment portions of the frame member and/or the at least one guiding member are/is configured to open or close an electric circuit of the medical imaging device, preferably to function as a limit switch or electrical contact.

The opening or closing of such an electric circuit may be required to operate the medical imaging device or particular functions thereof. The opening or closing of such electric circuit may provide, alternatively or in addition, an optic and/or acoustic indicator indicating a proper attachment of the sterile cover to the medical imaging device. For example, a light is turned on or off when the guiding member has closed or opened a respective electric circuit.

With the guiding member functioning as limit switch, the closing and/or opening of an electric circuit is linked to the final attachment position of the sterile cover. Accordingly, the functionality of a limit switch may support safety aspects. Alternatively or in addition, with the guiding member functioning as a electrical contact the closing of a contact or circuit is less prone to be a result of anything else but the electrical contact. For example, opening of a circuit may also occur due to a breakage of wires or the like. Instead, closing an electric circuit is less likely to happen unintentionally.

Advantageously, the transparent cover portion has a thickness in an imaging direction of 0,4 to 0,6 mm. Preferably, it has a refraction index of about 1,49.

Due to the combination of the thickness in the imaging direction when attached to a medical imaging device and a refraction index of substantially 1,49, the focus of the medical imaging device or the imaging components, respectively, may not be shifted significantly when the medical imaging device orbits a point of interest. With other words, the focus remains substantially constant at the point of interest during orbiting of the medical imaging device. Even though a control may compensate a focus shift by adapting the distance between the medical imaging device and the point of interest in dependence of the rotational angle, such control may become extremely complex.

A refraction index of substantially 1,49 may be achieved by using PMMA, i.e. polymethylmethacrylate, as material for the transparent cover portion. Increasing deviations from the defined refraction index in combination with the respective thickness of the transparent cover, impairs the control of the focus.

In other aspect, the present invention relates to a sterile medical imaging system, comprising: a medical imaging device and the sterile cover as described above, wherein an outer portion of the medical imaging device extends outwardly with a positive convergence angle from the imaging front side of the medical imaging device, and wherein the outer portion of the medical imaging device extending outwardly from the imaging front side of the medical imaging device comprises at least two attachment portions, preferably recessed in said outer portion, corresponding to the attachment portions of the sterile cover.

Accordingly, the attachment portions of the medical imaging device are configured to engage with corresponding attachment portions of the sterile cover to form a form-fit connection. Depending on the design of the attachment portions of the medical imaging device and/or sterile cover, the form-fit connection may be further supported by force-fitting elements. For example, a snap of a snap-fit member as attachment portion of a sterile cover may not only engage with a recess of a medical imaging device in terms of a form-fit connection. If the snap-member is still deformed elastically when arranged in a form-fit connection, the snaps may also apply a compressive force on the recess or the medical imaging device, respectively. Thus, the attachment may be secured more efficiently.

Furthermore, when the attachment portions of the medical imaging device are recessed in the outer portion of the medical imaging device, the number of potentially outwardly protruding contours and thus, interfering contours of the medical imaging device may be reduced.

Preferably, the positive convergence angle is equal to or less than 15°.

The positive convergence angle may correspond to a convergence angle of the optical components of the medical imaging device. Consequently, a compact medical imaging device may be provided. Furthermore, a limited convergence angle may also ease the design of the attachment portions of the sterile cover to engage with an attachment portion of the medical imaging device on an outer portion.

In some embodiments, the surface of transparent cover portion facing at least one imaging component forms an angle of about 84° to 87° with the optical axis of the at least one imaging component, wherein the distance between the surface of the transparent cover and the surface of the imaging component facing the transparent cover is preferably equal to or less than 25 mm.

The angle of 84° to 87° between the surface of the sterile cover facing the medical imaging device and the optical component of the medical imaging device also supports a sufficiently stabile focus, in particular, in further view of a thickness the transparent cover portion of the sterile cover and a refraction index of the transparent cover portion of substantially 1,49.

Furthermore, if the distance between the surface of the transparent cover and the surface of the imaging component facing the transparent cover is more than 25 mm, the amount of light scattered from the field of interest due to its illumination may impair the imaging by the imaging components of the medical imaging device. In other words, the amount of scattered light entering any imaging component may be sufficiently limited by restricting the distance between the surface of the transparent cover and the surface of the imaging component.

Preferably, the at least two attachment portions of the sterile cover and the at least two attachment portions of the medical imaging device, respectively, and/or the guiding member of the sterile cover are configured to provide only one attachment orientation.

The attachment portions may, for example, be positioned such that the sterile cover is only attachable in one orientation of the sterile cover with respect to the medical imaging device. This may be advantageous, if the transparent cover portion may only allow an unobstructed view through the imaging components in one orientation of the sterile cover. An unambiguous orientation of the sterile cover to be attached may also be provided, alternatively or in addition, by at least one guiding member and/or attachment portion with a protruding length that, for example, fits only in one corresponding recess of the medical imaging device to allow the attachment portions of the sterile cover to be moved into engagement with the attachment portions of the medical imaging device. In other words, any measure to reduce the likelihood of a misalignment is directed to allowing an attachment only in a defined orientation of the sterile cover with respect to the medical imaging device and/or at least restricts the functionality of the medical imaging device and/or indicates a misalignment in the event of an undesired positional relationship.

Advantageously, the medical imaging device comprises an electric circuit (13) closable by at least one of the at least two attachment portions of the sterile cover (30) and/or the at least one guiding member of the sterile cover.

As already described in the context of the sterile cover, such electric circuit may be used to indicate a predetermined attachment of the sterile cover to the medical imaging device. Further, the electric circuit may, alternatively or in addition to the above measures, only be opened or closed if the sterile cover is attached to the medical imaging device in a proper orientation.

In some embodiments, the medical imaging device of the sterile medical imaging system is a microscope.

Further advantages, aspects and details of the disclosure are subject to the claims, the following description of preferred embodiments applying the principles of the disclosure and drawings. In the figures, identical reference signs denote identical features and functions.

### Brief Description of the Drawings

- Fig. 1: is a schematic cross-section of a sterile cover and medical imaging device according to an exemplary embodiment;
- Fig. 2: is a schematic cross-section of the sterile cover and medical imaging system according to Fig. 1 when the sterile cover is attached to the medical imaging device;
- Fig. 3: is an enlarged view of the sterile cover and the region of the medical imaging device facing the sterile cover according to Figs. 1 and 2; and
- Fig. 4: is an exemplary view of a design of a guiding member and recess.

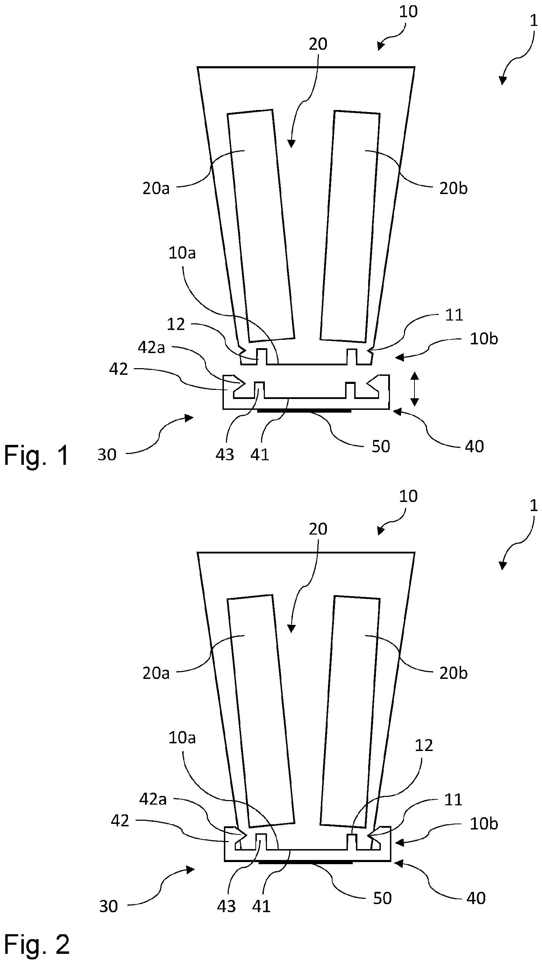

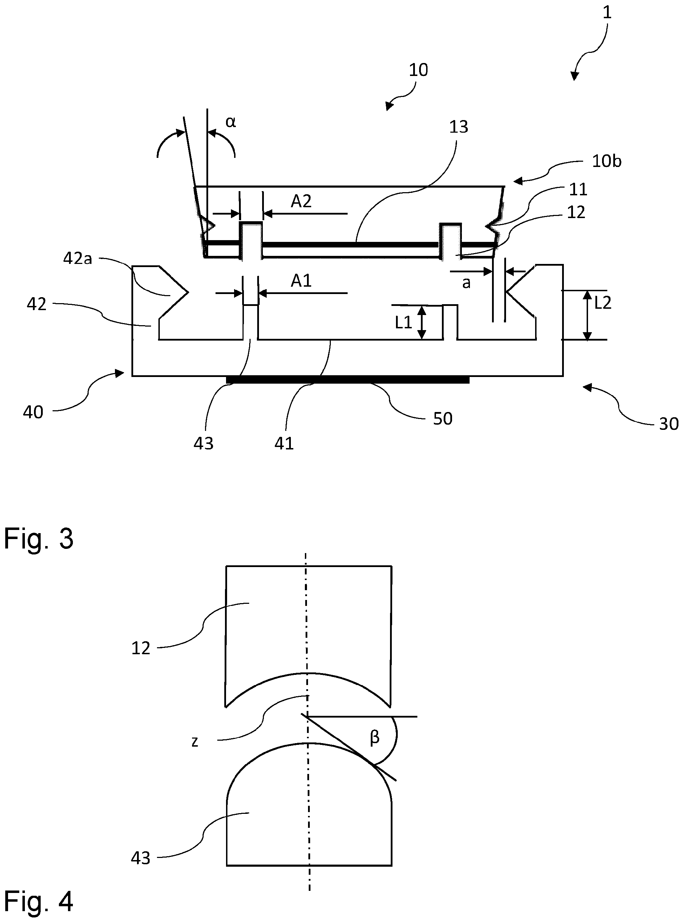

Fig. 1 is a schematic cross-section of a medical imaging device 10 and a sterile cover 30 of a sterile medical imaging system 1. The medical imaging device 10 comprises an imaging front side 10a facing the sterile cover 30 in an attachment orientation, in which the sterile cover 30 can be attached to or detached from the medical imaging device 10 by a bidirectional movement as indicated by the double arrow. An outer portion 10b of the medical imaging device 10 extends outwardly from the imaging front side 10a of the medical imaging device 10. The convergence angle α (Fig. 3) of the outer portion 10b is similar to the convergence angle of the imaging components 20a, 20b of an imaging unit 20. Here, each imaging component 20a, 20b is configured as an ocular, which provide an angle in-between thereof to be aligned on a common field of interest. Due to the similar convergence angle, the contour of the medical imaging device 10 may be designed compact. In the shown embodiment, the outer portion 10b also comprises two attachment portions 11 formed by notches to receive corresponding attachment portions of the sterile cover 30, as addressed later. Additionally, the imaging front side 10a provides two recesses 12 to allow the sterile cover 30 to be guided by moving corresponding guiding members 43 within the recesses 12. It should be noted that the number of attachment portions 11 and/or recesses 12 is not limited to the number as shown in the exemplary embodiment of Fig. 1. However, at least two of respective attachment portions 11 and/or recesses 12 provide at least some stability in terms of reducing the likelihood of the sterile cover to tilt without an excessive number of connection components. However, an increased number of attachment portions 11 and/or recesses 12 may provide more options to attach and/or guide different types of sterile covers 30 to a medical imaging device 10.

The sterile cover 30 comprises a frame member 40 and a transparent cover portion 50. The transparent cover portion 50 is affixed to the frame 40 and configured to allow the imaging unit 20 of the medical imaging device 10 to image a field of interest through the transparent cover portion 50 while being disposed in front of the medical imaging device 10 by the attachment of the frame member 40 to the medical imaging device 10. The frame member 40 provides a facing side 41 which is intended to face the imaging front side 10a of the medical imaging device 10 in an attachment orientation. The transparent cover portion 50 may be disposed in the frame member 40 with its surface opposite to the facing side 41 being flush with the frame surface also opposite to the facing side 41 to be disinfected or sterilized without interfering contours. Alternatively, such surface of the transparent cover portion 50 may be recessed within the frame member 40 to enhance the protection of the transparent cover portion 50.

The frame member 40 also comprises two attachment portions 42 intended to engage with the attachment portions 11 of the medical imaging device 10 to attach the sterile cover 30 to the medical imaging device 10. According to the illustrated embodiment, the attachment portions 42 are configured as snap-fit members protruding from the facing side 41 of the frame member 40 at opposite ends of the frame member 40. Here, the snap-fit members protrude perpendicular to the facing side 41. However, the attachment portions 42, irrespective of their specific design, may also protrude at an angle deviating from 90°, for example, according to design constraints. The attachment portions 42 configured as snap-members provide a snap at each respective end opposite to the facing side 41.

Further, the frame member 40 comprises to guiding members 43, which also protrude from the facing side 41. As per the guiding function, the guiding members 43 protrude substantially perpendicular to the facing side 41. However, slight deviations may be possible according to specific design options.

Fig. 2 shows the sterile cover 30 attached to the medical imaging device 1 in the same cross-section as per Fig. 1. The sterile cover 30 is attached by a relative movement to reduce the distance between the facing side 41 and the imaging front side 10a. Accordingly, the attachment portions 42 as resilient snap-fit members with snaps 42a are elastically deformed over at least some extension of the outer portion 10b before arriving at the corresponding attachment portions 11. When the snaps 42a arrive at attachment portions 11 formed as notches in this exemplary embodiment, the snaps 42a are forced into the notches due the elastic restoring forces of the resilient attachment portions 42.

The imaging components 20a, 20b of the imaging unit 20 or their optical axis, respectively, are each inclined by 5° with respect to a vertical axis in Fig. 2. In other words, the imaging components 20a, 20b provide a convergence angle of 10° between each other. Accordingly, the optical axis of each imaging component 20a, 20b forms an angle of 85° with the transparent cover portion 50. Such angle is preferably between 84° and 87°. To reduce a focus shift in such configuration to an acceptable minimum, the transparent cover portion has a thickness in direction of the optical axis of the respective imaging component 20a, 20b of 0,5 mm +/- 0,1 mm, i.e. between 0,4 mm and 0,6 mm. Further, the refraction index is about 1,49 with PMMA selected as material for the transparent cover portion 50. To reduce the amount of scattered light entering the imaging components 20a, 20b, the transparent cover portion 50 is distanced less than 25 mm from each imaging component 20a, 20b. The size of the transparent cover portion 30 in this embodiment is limited to the size required for imaging. The amount of scattered light entering the imaging components may therefore be optimized by the combination of the distance and sized of the transparent cover portion 50.

Fig. 3 is an enlarged view of the sterile cover 30 and the region of the medical imaging device 10 facing the sterile cover 30 according to Figs. 1 and 2. The guiding members 43 provide a protruding length L1. However, the guiding members 43 may also provide different hides. For example, if one guiding member 43 provides a greater length L1 + ΔL and the medical imaging device 10 comprises a corresponding recess, the sterile cover 30 may only be able to be attached, if each guiding member 43 enters its corresponding recess 12. Assuming that the corresponding recesses 12 have a depth of L1 and L1 + ΔL, respectively, the length of the protruding length L2 for engagement should be at least L1 minus the difference in length ΔL, i.e. L2min = L1 - ΔL. Thus, the sterile cover 30 may only be attached in one attachment orientation. In other words, the attachment portions 42 cannot engage with the attachment portions 11 when the sterile cover is, for example, turned around a vertical axis in Fig. 3 by 180°.

Irrespective of different lengths of the guiding members 43 and depths of the recesses 12, the protruding length L2 for engagement of the attachment portions 42 in the embodiment of Fig. 1 is greater than the protruding length of the guiding members 43. Accordingly, a ratio between l1 and L2 is less than 1, preferably between 0,41 and 0,74. Furthermore, the ratio of the cross-section A1 of the guiding member 43 perpendicular to its protruding length L1 to be received by the corresponding recess 12 to the cross-section A2 of said recess 11 perpendicular to its depth is also less than 1, preferable in general between 0,15 and 0,9. In the embodiment according to Fig. 3 the medical imaging device 10 comprises an electric circuit 13 that is closed when the sterile cover 30 is attached to the medical imaging device 10 due to engagement of the attachment portions 42, 11. In such embodiment, the ratio of A1 to A2 should be rather in a range of 0,9 than in a range of 0,15 to ensure closing of the electric circuit 13. On the other hand, a lower ratio may be less sensitive to manufacturing tolerance that may result in frictional forces or otherwise impede the insertion of the guiding members 43 in the recesses 12. To allow a low ration A1 to A2 in combination with the electric circuit 13, the electric circuit may form a contact area at the bottom of the recesses 12. The contacts can be closed by the end surfaces of the guiding members 43. Thus, such configuration is less sensitive to lateral tolerances but to tolerance in corresponding lengths. To reduce tolerance in length, the end surfaces of the guiding members and/or the bottom of the recesses 11 may comprise resilient surface portions to compensate potential tolerances. The combination of the guiding members 43 and recesses 12 in view of closing or opening an electric circuit may also be configured as e mechanical switch, e.g. a button or actuator disposed in a corresponding recess 12 that is movable in the insertion direction of the guiding member 43 to close or open an electric circuit. Even though the embodiment of Fig. 3 illustrates an example of an electric circuit 13 to be closed by the two guiding members 43, the electric circuit 13 may also be configured to be closed by at least one of the guiding members 43. The same applies vice versa in the event of an electric circuit to be opened.

Further, the attachment portions 42 are configured to provide a gap a between respective snaps 42a of the snap-fit members as attachment portions 42 and the imaging front side 10a of the medical imaging device 10 in parallel to the facing side 41 of the frame member 40, here less than 4 mm, when the medical imaging device 10 extends outwardly with a positive convergence angle α from the imaging front side 10a. The convergence angle α in the exemplary embodiment is 5°. Due to the gap a, the resilient attachment members 42 are not elastically deformed when initially moved over the imaging front side 10a but during the course of the movement to approach the corresponding attachment portions 11 of the medical imaging device 10. Thus, the elastic deformation may be reduced to a minimum to ensure an elastic deformation required for the snaps 42a to be moved in a locking position into the notches as attachment portions 11 due to the elastic restoring forces. However, the gap a should still be restricted to reduces the protruding length L2 for engagement under consideration of the convergence angle α, here 5°.

Fig. 4 is an exemplary view of a design of a guiding member 43 and a corresponding recess 12. The end surface of the guiding member 43 facing the bottom surface of the recess 12 is tapered and forms a concave surface with an inclination angle β with respect to a plane perpendicular with the longitudinal axis z. Preferably, the inclination angle β is between 20° and 45°, which may also provide a centering functionality. Alternatively to the concave surface the end surface of the guiding member may be conical. However, a concave shape with respect to a radius near the longitudinal axis z may provide manufacturing advantages. The bottom surface of the recess 12 provides a corresponding surface shape in term of a positive-negative configuration. Due to such design, said combination is particularly advantageous, if the guiding member 43 is intended to actuate the bottom surface of the recess 12 as an actuator to close or open, for example, the electric circuit 13. Specifically, the actuator is also actuated by the guiding member 43 even if such actuation takes place under bending or skewed actuation.

It is to be noted that the given examples are specific embodiments and not intended to restrict the scope of protection given in the claims. In particular, single features of one embodiment may be combined with another embodiment as long as such combination is not reasonably excluded. As an example, the electric circuit to be opened or closed may be provided, alternatively or in addition, by the sterile cover.

### List of reference signs

- 1: sterile medical imaging system
- 10: medical imaging device
- 10a: imaging front side
- 10b: outer portion
- 11: attachment portion (medical imaging device 10)
- 12: recess
- 13: electric circuit
- 20: imaging unit
- 20a: imaging component
- 20b: imaging component
- 30: sterile cover
- 40: frame member
- 41: facing side
- 42: attachment portion (sterile cover 30)
- 42a: snap
- 43: guiding member
- 50: transparent cover portion
- a: gap
- A1: cross-section (guiding member 43)
- A2: cross-section (recess 12)
- L1: length (guiding member 43)
- L2: length for engagement (attachment portion 42)
- z: longitudinal axis
- α: convergence angle
- β: inclination angle

## Claims

1. Sterile cover (30) for a medical imaging device (10), comprising:
a frame member (40) configured to cover an imaging front side (10a) of the medical imaging device (10), wherein a side intended to face the imaging front side (10a) of the medical device (10) is a facing side (41) of the frame member (40), and
at least one transparent cover portion (50) within the frame member (40) arranged to allow at least imaging by the medical imaging device (10) when the frame member (40) is attached to the medical imaging device (10), wherein
the frame member (40) comprises at least two attachment portions (42) configured to provide a positive-fit connection with corresponding attachment portions (11) of the medical imaging device (10), and
wherein the frame member (40) further comprises at least one guiding member (43) protruding from the facing side (41) of the frame member (40) intended to be received by a recess (12) on the imaging front side (10a) of the medical imaging device (10).

2. The sterile cover (30) according to claim 1, wherein the at least two attachment portions (42) are provided on two opposing positions on the facing side (41) of the frame member (40).

3. The sterile cover (30) according to claim 1 or 2, wherein the at least two attachment portions (42) are configured as resilient snap-fit members, preferably protruding from a periphery of the frame member in a direction extending from the facing side (41) of the frame member (40).

4. The sterile cover (30) according to claim 3, wherein snap-fit members are configured to provide a gap (a) between respective snaps (42a) of the snap-fit members and the imaging front side (10a) of the medical imaging device (10) in parallel to the facing side (41) of the frame member (40), preferably less than 4 mm, when the medical imaging device (10) extends outwardly with a positive convergence angle (α) from the imaging front side (10a).

5. The sterile cover according to any one of the preceding claims, wherein a ratio of a protruding length (L1) of the at least one guiding member (43) and a protruding length (L2) for engagement of the snap-fit members according to claim 3 or 4 protruding from the facing side (41) of the frame member (40) to the respective ends of snaps (42a) opposite to the facing side (1 0a) is less than 1, preferably between 0,41 and 0,74.

6. The sterile cover (30) according to any one of the preceding claims, wherein a ratio of a cross section (A1) perpendicular to the protruding length (L1) of the at least one guiding member (43) and a corresponding cross-section (A2) of the recess (12) on the imaging front side (10a) of the medical imaging device (10) is less than 1, preferably between 0,15 and 0,9.

7. The sterile cover (30) according to any one of the preceding claims, wherein at least one of the at least two attachment portions (42) of the frame member (40) and/or the at least one guiding member (43) are/is configured to open or close an electric circuit (13) of the medical imaging device (10), preferably to function as a limit switch or to establish an electrical contact.

8. The sterile cover according to any one of the preceding claims, wherein the transparent cover portion (50) has a thickness in an imaging direction of 0,4 to 0,6 mm and/or a refraction index of about 1,49.

9. Sterile medical imaging system (1), comprising:
a medical imaging device (10) and
a sterile cover (30) according to any one of the claims 1 to 8,
wherein an outer portion (10b) of the medical imaging device (10) extends outwardly with a positive convergence angle (α) from the imaging front side (10a) of the medical imaging device (10), and
wherein the outer portion (10b) of the medical imaging device (10) extending outwardly from the imaging front side (10a) of the medical imaging device (10) comprises at least two attachment portions (11), preferably recessed in said outer portion (10b), corresponding to the attachment portions (42) of the sterile cover (30).

10. The sterile medical imaging system (1) according to claim 9, wherein the positive convergence angle (α) is equal to or less than 15°.

11. The sterile medical imaging system (1) according to claim 9 or 10, wherein the surface of transparent cover portion (50) facing at least one imaging component (20a, 20b) forms an angle of about 84° to 87° with the optical axis of the at least one imaging component (20a, 20b), wherein the distance between the surface of the transparent cover (50) and the surface of the imaging component (20a, 20b) facing the transparent cover (50) is preferably equal to or less than 25 mm.

12. The sterile medical imaging system (1) according to any one of the claims 9 to 11, wherein the at least two attachment portions (42) of the sterile cover (30) and the at least two attachment portions (11) of the medical imaging device (10), respectively, and/or the guiding member (43) of the sterile cover (30) are configured to provide only one attachment orientation.

13. The sterile medical imaging system (1) according to any one of the claims 9 to 12, wherein the medical imaging device (10) comprises an electric circuit (13) closable by at least one of the at least two attachment portions (42) of the sterile cover (30) and/or the at least one guiding member (43) of the sterile cover (30).

14. The sterile medical imaging system (1) according to any one of the claims 9 to 13, wherein the medical imaging device (10) is a microscope.

## Patentansprüche

1. Sterilabdeckung (30) für eine medizinische Bildgebungsvorrichtung (10), umfassend:
ein Rahmenelement (40), das konfiguriert ist, um eine Bildgebungsvorderseite (10a) der medizinischen Bildgebungsvorrichtung (10) abzudecken, wobei eine Seite, die dazu vorgesehen ist, der Bildgebungsvorderseite (10a) der medizinischen Vorrichtung (10) zugewandt zu sein, eine zugewandte Seite (41) des Rahmenelements (40) ist, und
mindestens einen transparenten Abdeckungsabschnitt (50) innerhalb des Rahmenelements (40), der angeordnet ist, um mindestens eine Bildgebung durch die medizinische Bildgebungsvorrichtung (10) zu ermöglichen, wenn das Rahmenelement (40) an der medizinischen Bildgebungsvorrichtung (10) befestigt ist,
wobei das Rahmenelement (40) mindestens zwei Befestigungsabschnitte (42) umfasst, die konfiguriert sind, um eine formschlüssige Verbindung mit entsprechenden Befestigungsabschnitten (11) der medizinischen Bildgebungsvorrichtung (10) bereitzustellen, und
wobei das Rahmenelement (40) ferner mindestens ein Führungselement (43) umfasst, das von der zugewandten Seite (41) des Rahmenelements (40) vorsteht, das dazu vorgesehen ist, von einer Aussparung (12) auf der Bildgebungsvorderseite (10a) der medizinischen Bildgebungsvorrichtung (10) aufgenommen zu werden.

2. Die Sterilabdeckung (30) nach Anspruch 1, wobei die mindestens zwei Befestigungsabschnitte (42) an zwei gegenüberliegenden Positionen auf der zugewandten Seite (41) des Rahmenelements (40) vorgesehen sind.

3. Die Sterilabdeckung (30) nach Anspruch 1 oder 2, wobei die mindestens zwei Befestigungsabschnitte (42) als elastische Schnappverbindungselemente konfiguriert sind, die vorzugsweise von einem Umfang des Rahmenelements in eine Richtung vorstehen, die sich von der zugewandten Seite (41) des Rahmenelements (40) erstreckt.

4. Die Sterilabdeckung (30) nach Anspruch 3, wobei Schnappverbindungselemente konfiguriert sind, um einen Spalt (a) zwischen jeweiligen Schnapphaken (42a) der Schnappverbindungselemente und der Bildgebungsvorderseite (10a) der medizinischen Bildgebungsvorrichtung (10) parallel zu der zugewandten Seite (41) des Rahmenelements (40) bereitzustellen, vorzugsweise weniger als 4 mm, wenn sich die medizinische Bildgebungsvorrichtung (10) mit einem positiven Konvergenzwinkel (α) von der Bildgebungsvorderseite (10a) nach außen erstreckt.

5. Die Sterilabdeckung nach einem der vorhergehenden Ansprüche, wobei ein Verhältnis einer vorstehenden Länge (L1) des mindestens einen Führungselements (43) und einer vorstehenden Länge (L2) für den Eingriff der Schnappverbindungselemente nach Anspruch 3 oder 4, die von der zugewandten Seite (41) des Rahmenelements (40) vorstehen, zu den jeweiligen Enden der Schnapphaken (42a) gegenüber der zugewandten Seite (10a) kleiner als 1 ist, vorzugsweise zwischen 0,41 und 0,74.

6. Die Sterilabdeckung (30) nach einem der vorhergehenden Ansprüche, wobei ein Verhältnis eines Querschnitts (A1) senkrecht zu der vorstehenden Länge (L1) des mindestens einen Führungselements (43) und eines entsprechenden Querschnitts (A2) der Aussparung (12) auf der Bildgebungsvorderseite (10a) der medizinischen Bildgebungsvorrichtung (10) kleiner als 1 ist, vorzugsweise zwischen 0,15 und 0,9.

7. Die Sterilabdeckung (30) nach einem der vorhergehenden Ansprüche, wobei mindestens einer der mindestens zwei Befestigungsabschnitte (42) des Rahmenelements (40) und/oder das mindestens eine Führungselement (43) konfiguriert ist/sind, um einen elektrischen Schaltkreis (13) der medizinischen Bildgebungsvorrichtung (10) zu öffnen oder zu schließen, vorzugsweise um als ein Endschalter zu fungieren oder um einen elektrischen Kontakt herzustellen.

8. Die Sterilabdeckung nach einem der vorhergehenden Ansprüche, wobei der transparente Abdeckungsabschnitt (50) eine Dicke in einer Bildgebungsrichtung von 0,4 bis 0,6 mm und/oder einen Brechungsindex von etwa 1,49 aufweist.

9. Steriles medizinisches Bildgebungssystem (1), umfassend:
eine medizinische Bildgebungsvorrichtung (10) und
eine Sterilabdeckung (30) nach einem der Ansprüche 1 bis 8,
wobei sich ein äußerer Abschnitt (10b) der medizinischen Bildgebungsvorrichtung (10) mit einem positiven Konvergenzwinkel (α) von der Bildgebungsvorderseite (10a) der medizinischen Bildgebungsvorrichtung (10) nach außen erstreckt, und
wobei der äußere Abschnitt (10b) der medizinischen Bildgebungsvorrichtung (10), der sich von der Bildgebungsvorderseite (10a) der medizinischen Bildgebungsvorrichtung (10) nach außen erstreckt, mindestens zwei Befestigungsabschnitte (11) umfasst, die vorzugsweise in den äußeren Abschnitt (10b) vertieft sind, entsprechend den Befestigungsabschnitten (42) der Sterilabdeckung (30).

10. Das sterile medizinische Bildgebungssystem (1) nach Anspruch 9, wobei der positive Konvergenzwinkel (α) gleich oder kleiner als 15° ist.

11. Das sterile medizinische Bildgebungssystem (1) nach Anspruch 9 oder 10, wobei die Oberfläche des transparenten Abdeckungsabschnitts (50), die mindestens einer Bildgebungskomponente (20a, 20b) zugewandt ist, einen Winkel von etwa 84° bis 87° mit der optischen Achse der mindestens einen Bildgebungskomponente (20a, 20b) bildet, wobei der Abstand zwischen der Oberfläche der transparenten Abdeckung (50) und der Oberfläche der Bildgebungskomponente (20a, 20b), die der transparenten Abdeckung (50) zugewandt ist, vorzugsweise gleich oder kleiner als 25 mm ist.

12. Das sterile medizinische Bildgebungssystem (1) nach einem der Ansprüche 9 bis 11, wobei die mindestens zwei Befestigungsabschnitte (42) der Sterilabdeckung (30) bzw. die mindestens zwei Befestigungsabschnitte (11) der medizinischen Bildgebungsvorrichtung (10) und/oder das Führungselement (43) der Sterilabdeckung (30) konfiguriert sind, um nur eine Befestigungsausrichtung bereitzustellen.

13. Das sterile medizinische Bildgebungssystem (1) nach einem der Ansprüche 9 bis 12, wobei die medizinische Bildgebungsvorrichtung (10) einen elektrischen Schaltkreis (13) umfasst, der durch mindestens einen der mindestens zwei Befestigungsabschnitte (42) der Sterilabdeckung (30) und/oder das mindestens eine Führungselement (43) der Sterilabdeckung (30) schließbar ist.

14. Das sterile medizinische Bildgebungssystem (1) nach einem der Ansprüche 9 bis 13, wobei die medizinische Bildgebungsvorrichtung (10) ein Mikroskop ist.

## Revendications

1. Un couvercle stérile (30) pour un dispositif d'imagerie médicale (10), comprenant :
un élément de cadre (40) configuré pour recouvrir un côté avant de prise de vue (10a) du dispositif d'imagerie médicale (10), dans lequel un côté destiné à faire face au côté avant de prise de vue (10a) du dispositif médical (10) est un côté en regard (41) de l'élément de cadre (40), et
au moins une portion de couvercle transparente (50) à l'intérieur de l'élément de cadre (40) agencée pour permettre au moins une prise de vue par le dispositif d'imagerie médicale (10) lorsque l'élément de cadre (40) est fixé au dispositif d'imagerie médicale (10), dans lequel
l'élément de cadre (40) comprend au moins deux portions de fixation (42) configurées pour fournir une connexion par ajustement positif avec des portions de fixation correspondantes (11) du dispositif d'imagerie médicale (10), et
dans lequel l'élément de cadre (40) comprend en outre au moins un élément de guidage (43) faisant saillie du côté en regard (41) de l'élément de cadre (40) destiné à être reçu par un évidement (12) sur le côté avant de prise de vue (10a) du dispositif d'imagerie médicale (10).

2. Le couvercle stérile (30) selon la revendication 1, dans lequel les au moins deux portions de fixation (42) sont prévues sur deux positions opposées sur le côté en regard (41) de l'élément de cadre (40).

3. Le couvercle stérile (30) selon la revendication 1 ou 2, dans lequel les au moins deux portions de fixation (42) sont configurées comme des éléments d'encliquetage élastiques, faisant de préférence saillie d'une périphérie de l'élément de cadre dans une direction s'étendant depuis le côté en regard (41) de l'élément de cadre (40).

4. Le couvercle stérile (30) selon la revendication 3, dans lequel des éléments d'encliquetage sont configurés pour fournir un espace (a) entre des encliquetages respectifs (42a) des éléments d'encliquetage et le côté avant de prise de vue (10a) du dispositif d'imagerie médicale (10) parallèlement au côté en regard (41) de l'élément de cadre (40), de préférence inférieur à 4 mm, lorsque le dispositif d'imagerie médicale (10) s'étend vers l'extérieur avec un angle de convergence positif (α) depuis le côté avant de prise de vue (10a).

5. Le couvercle stérile selon l'une quelconque des revendications précédentes, dans lequel un rapport d'une longueur en saillie (L1) de l'au moins un élément de guidage (43) et d'une longueur en saillie (L2) pour l'engagement des éléments d'encliquetage selon la revendication 3 ou 4 faisant saillie du côté en regard (41) de l'élément de cadre (40) aux extrémités respectives des encliquetages (42a) opposés au côté en regard (10a) est inférieur à 1, de préférence entre 0,41 et 0,74.

6. Le couvercle stérile (30) selon l'une quelconque des revendications précédentes, dans lequel un rapport d'une section transversale (A1) perpendiculaire à la longueur en saillie (L1) de l'au moins un élément de guidage (43) et d'une section transversale correspondante (A2) de l'évidement (12) sur le côté avant de prise de vue (10a) du dispositif d'imagerie médicale (10) est inférieur à 1, de préférence entre 0,15 et 0,9.

7. Le couvercle stérile (30) selon l'une quelconque des revendications précédentes, dans lequel au moins l'une des au moins deux portions de fixation (42) de l'élément de cadre (40) et/ou de l'au moins un élément de guidage (43) est/sont configurée(s) pour ouvrir ou fermer un circuit électrique (13) du dispositif d'imagerie médicale (10), de préférence pour fonctionner comme un interrupteur de limitation ou pour établir un contact électrique.

8. Le couvercle stérile selon l'une quelconque des revendications précédentes, dans lequel la portion de couvercle transparente (50) a une épaisseur dans une direction d'imagerie de 0,4 à 0,6 mm et/ou un indice de réfraction d'environ 1,49.

9. Un système d'imagerie médicale stérile (1), comprenant :
un dispositif d'imagerie médicale (10) et
un couvercle stérile (30) selon l'une quelconque des revendications 1 à 8,
dans lequel une portion extérieure (10b) du dispositif d'imagerie médicale (10) s'étend vers l'extérieur avec un angle de convergence positif (α) depuis le côté avant de prise de vue (10a) du dispositif d'imagerie médicale (10), et
dans lequel la portion extérieure (10b) du dispositif d'imagerie médicale (10) s'étendant vers l'extérieur depuis le côté avant de prise de vue (10a) du dispositif d'imagerie médicale (10) comprend au moins deux portions de fixation (11), de préférence en retrait dans ladite portion extérieure (10b), correspondant aux portions de fixation (42) du couvercle stérile (30).

10. Le système d'imagerie médicale stérile (1) selon la revendication 9, dans lequel l'angle de convergence positif (α) est égal ou inférieur à 15°.

11. Le système d'imagerie médicale stérile (1) selon la revendication 9 ou 10, dans lequel la surface de la portion de couvercle transparente (50) faisant face à au moins un composant d'imagerie (20a, 20b) forme un angle d'environ 84° à 87° avec l'axe optique de l'au moins un composant d'imagerie (20a, 20b), dans lequel la distance entre la surface du couvercle transparent (50) et la surface du composant d'imagerie (20a, 20b) faisant face au couvercle transparent (50) est de préférence égale ou inférieure à 25 mm.

12. Le système d'imagerie médicale stérile (1) selon l'une quelconque des revendications 9 à 11, dans lequel les au moins deux portions de fixation (42) du couvercle stérile (30) et les au moins deux portions de fixation (11) du dispositif d'imagerie médicale (10), respectivement, et/ou l'élément de guidage (43) du couvercle stérile (30) sont configurés pour fournir une seule orientation de fixation.

13. Le système d'imagerie médicale stérile (1) selon l'une quelconque des revendications 9 à 12, dans lequel le dispositif d'imagerie médicale (10) comprend un circuit électrique (13) pouvant être fermé par au moins l'une des au moins deux portions de fixation (42) du couvercle stérile (30) et/ou de l'au moins un élément de guidage (43) du couvercle stérile (30).

14. Le système d'imagerie médicale stérile (1) selon l'une quelconque des revendications 9 à 13, dans lequel le dispositif d'imagerie médicale (10) est un microscope.
